# EUROPEAN PATENT APPLICATION

(11) **EP 4 776 030 A1**
(43) Date of publication of application: **15.07.2026**
(21) Application number: 25225342.2
(22) Date of filing: 18.12.2025
(51) Int. Cl.: G01T 1/161, G01T 1/24, H10F 39/18

(54) **SYSTEM AND METHOD FOR LEAKAGE CURRENT REDUCTION IN AN X-RAY DETECTOR**

(30) Priority: 09.01.2025 US 202519014586
(71) Applicant: GE Precision Healthcare LLC, Waukesha, WI 53188 (US)
(72) Inventor: JACOB, Biju, Niskayuna, 12309-1027 (US); HENNESSY, William Andrew, Niskayuna, 12309-1027 (US); KONKLE, Nicholas Ryan, Waukesha, 53188 (US); CAMPANINI, Donato, Waukesha, 53188 (US)
(74) Representative: AWA Sweden AB

(57) **Abstract**

A radiation imaging apparatus (90) includes a substrate (92) and a radiation-sensitive imaging region (96) in the substrate (92) including pixels (60). The radiation imaging apparatus (90) includes a guard region (94) at or immediately adjacent a cut edge (95) of the substrate (92), wherein the guard region (94) reduces the leakage current reaching the radiation-sensitive imaging region (96) from the cut edge (95) when the radiation imaging apparatus (90) is in use. The radiation-sensitive imaging region (96) is electrically reverse biased with respect to the substrate (92). The radiation-sensitive imaging region (96) includes a portion (134) adjacent the guard region (94) that is closest to where incident radiation impacts the radiation imaging apparatus (90). The pixels (60) include a first pixel electrode (146) located in the portion (134) where a first longitudinal end (150) of the first pixel electrode (146) closest to the guard region (94) is a first distance (154) from the guard region (94) that reduces an amount of the leakage current received by the first pixel electrode (146).

## Description

### BACKGROUND

The subject matter disclosed herein relates to imaging systems and, more particularly, to a system and method for leakage current reduction in an X-ray detector.

Non-invasive imaging technologies allow images of the internal structures or features of a subject (patient, manufactured good, baggage, package, or passenger) to be obtained without physical contact.

For example, in X-ray-based imaging technologies, X-ray radiation penetrates a subject of interest, such as a human patient, and a portion of the radiation impacts a detector where the intensity data is collected. In digital X-ray systems, a detector produces signals representative of the amount or intensity of radiation impacting discrete pixel regions of a detector surface. The signals may then be processed to generate an image that may be displayed for review.

In one such X-ray based technique, known as computed tomography (CT), a scanner may project fan-shaped or cone-shaped X-ray beams from an X-ray source at numerous view angle positions about an object being imaged, such as a patient. The X-ray beams are attenuated as they traverse the object and are detected by a set of detector elements which produce signals representing the intensity or number of incident X-rays reaching the detector. The signals are processed to produce data representing the line integrals of the linear attenuation coefficients of the object along the X-ray paths. These signals are typically called "projection data" or just "projections". By using reconstruction techniques, such as filtered backprojection, images may be generated that represent a cross sectional slice or three-dimensional (3D) volume of a region of interest of the patient or imaged object. In a medical context, pathologies or other structures of interest may then be located or identified from the reconstructed images or rendered volume.

Some CT detectors include photon counting detectors. A photon counting detector converts each detected X-ray photon in the energy unit (keV) into a voltage pulse in the pulse height unit (mV). An X-ray photon is absorbed in a semiconductor material (e.g., cadmium zinc telluride (CZT), silicon, etc.) resulting in generation of photocharge proportional to the X-ray photon energy. A photodiode or diode, separates the electron-hole pairs and generates a current pulse at its output. The current is fed into application-specific integrated circuit (ASIC), which tracks individual current pulses, determines the energy of the X-ray photons that generated these pulses and assigns them to the appropriate energy bins.

In the state-of-the-art photon counting detectors, the semiconductor sensor consists of an array of photodiode pixels (e.g., diodes). Leakage (dark) current in the pixels needs to be very low to ensure acceptable image quality. Several factors, including material quality, semiconductor processing, wafer dicing, and so forth can cause high leakage current. Only a few pixel diodes with high leakage current can be tolerated. Pixel diodes at the edge of the sensor (especially the portion closest to incident radiation impacting the detector) are prone to high leakage in failing sensors. This may lead to several wafer batches being discarded in production due to these high leakage diodes which significantly impacts the detector cost.

### SUMMARY

Certain embodiments commensurate in scope with the originally claimed subject matter are summarized below. These embodiments are not intended to limit the scope of the claimed subject matter, but rather these embodiments are intended only to provide a brief summary of possible forms of the subject matter. Indeed, the subject matter may encompass a variety of forms that may be similar to or different from the embodiments set forth below.

In one embodiment, a radiation imaging apparatus is provided. The radiation imaging apparatus includes a substrate. The radiation imaging apparatus includes at least one radiation-sensitive imaging region in the substrate including a plurality of pixels, wherein each pixel of the plurality of pixels is configured to act as a detector element. The radiation imaging apparatus also includes a guard region at or immediately adjacent a cut edge of the substrate, wherein the guard region collects leakage current from the cut edge and reduces leakage current reaching the at least one radiation-sensitive imaging region from the cut edge when the radiation imaging apparatus is in use. The at least one radiation-sensitive imaging region is electrically reverse biased with respect to the substrate. The at least one radiation-sensitive imaging region includes a portion adjacent the guard region that is closest to where incident radiation impacts the radiation imaging apparatus. The plurality of pixel electrodes includes a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance.

In another embodiment, a method of reducing leakage current in a radiation imaging apparatus is provided. The method includes providing a substrate with at least one radiation-sensitive imaging region therein, wherein the at least one radiation-sensitive imaging region includes a plurality of pixels, wherein each pixel of the plurality of pixels is configured to act as a detector element. The method also includes forming a guard region in the substrate at or immediately adjacent a cut edge of the substrate to collect leakage current from the cut edge and to reduce leakage current reaching the at least one radiation-sensitive imaging region from the cut edge when the radiation imaging apparatus is in use. The method further includes electrically reverse biasing the at least one radiation-sensitive imaging region relative to the substrate. The at least one radiation-sensitive imaging region includes a portion adjacent the guard region that is closest to where incident radiation impacts the radiation imaging apparatus. The plurality of pixel electrodes includes a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance.

In a further embodiment, a photon-counting X-ray detector is provided. The photon counting detector includes a plurality of detector sub-modules. Each detector sub-module includes a semiconductor layer. Each detector sub-module also includes at least one radiation-sensitive imaging region in the semiconductor layer, wherein the at least one radiation-sensitive imaging region includes a plurality of pixels, wherein each pixel of the plurality of pixels is configured to act as a detector element. Each detector sub-module further includes a guard region at or immediately adjacent a cut edge of the semiconductor layer, wherein the guard region collects leakage current from the cut edge and reduces leakage current reaching the at least one radiation-sensitive imaging region from the cut edge when the radiation imaging apparatus is in use. The at least one radiation-sensitive imaging region is electrically reverse biased with respect to the semiconductor layer. The at least one radiation-sensitive imaging region includes a portion adjacent the guard region that is closest to where incident radiation impacts the radiation imaging apparatus. The plurality of pixel electrodes includes a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance.

### BRIEF DESCRIPTION OF THE DRAWINGS

These and other features, aspects, and advantages of the disclosed subject matter will become better understood when the following detailed description is read with reference to the accompanying drawings in which like characters represent like parts throughout the drawings, wherein:
FIGS. 1A and 1B are a pictorial view and a block diagram representation, respectively, of a CT system, in accordance with aspects of the present disclosure;
FIG. 2 is a schematic diagram illustrating an example of modular X-ray detector sub-modules arranged side-by-side and stacked one after the other, in accordance with aspects of the present disclosure;
FIG. 3 is a cross-sectional view through a portion of an X-ray detector sub-module, in accordance with aspects of the present disclosure;
FIG. 4 is a schematic diagram of a sensor illustrating leakage current physics;
FIG. 5 is a schematic diagram of different configurations of a sensor illustrating an alteration in pixel electrode position to reduce leakage current, in accordance with aspects of the present disclosure;
FIG. 6 is a schematic diagram of a sensor comparing different pixel electrode positions relative to a guard ring, in accordance with aspects of the present disclosure;
FIG. 7 is a schematic diagram comparing X-ray detectors having a top row of electrodes in a different positions relative to a guard ring, in accordance with aspects of the present disclosure;
FIG. 8 is a table depicting simulated leakage current for increasing pixel electrode to guard ring distance, in accordance with aspects of the present disclosure;
FIG. 9 is a table depicting simulated breakdown voltage for increasing pixel electrode to guard ring distance, in accordance with aspects of the present disclosure; and
FIG. 10 is a flow chart of a method for reducing leakage current in a radiation imaging apparatus.

### DETAILED DESCRIPTION

One or more specific embodiments will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present subject matter, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be non-limiting, and thus additional numerical values, ranges, and percentages are within the scope of the disclosed embodiments.

While aspects of the following discussion are provided in the context of medical imaging, it should be appreciated that the disclosed techniques are not limited to such medical contexts. Indeed, the provision of examples and explanations in such a medical context is only to facilitate explanation by providing instances of real-world implementations and applications. However, the disclosed techniques may also be utilized in other contexts, such as image reconstruction for non-destructive inspection of manufactured parts or goods (i.e., quality control or quality review applications), and/or the non-invasive inspection of packages, boxes, luggage, and so forth (i.e., security or screening applications). In general, the disclosed techniques may be useful in any imaging or screening context or image processing or photography field where a set or type of acquired data undergoes a reconstruction process to generate an image or volume.

Energy-resolved, photon counting detectors can provide spectral information that is not available with conventional energy-integrating detectors. One type of energy-discriminating, photon counting detection technology employs silicon strips as a direct-conversion sensor material. Use of silicon as the direct-conversion material may provide a higher count rate capability than may be obtained with other direct-conversion materials, such as CZT or CdTe. In certain embodiments, the detector may be arranged edge-on to increase absorption efficiency by enabling an absorption depth to be chosen to any length and the detector can still be fully depleted without going to very high voltages. The detector elements on detector sub-modules or sensors may include detector elements (in particular those detector elements along an edge of the detector sub-module or sensor where the incident radiation first encounters the detector sub-module) that are prone to high leakage current.

The present approaches mitigate this problem by providing systems and methods for leakage current reduction in an X-ray detector. In particular, pixels (e.g., diodes) at the edge of the sensor (e.g., detector sub-module) closest to interaction with incident radiation, which are typically prone to high leakage, are shortened (along their longitudinal length) so that these edge pixels are farther away from a current capture guard electrode to reduce an amount of the leakage current received by each of these edge pixels. This reduction in the leakage current in the edge pixels does not impact detection efficiency and does not impact sensor reliability (i.e., impact on breakdown voltage is negligible). The disclosed embodiments improve sensor yield resulting in detector cost savings. The disclosed embodiments will reduce dark counts due to a lower leakage current and thus improve image quality.

In certain embodiments, a radiation imaging apparatus includes a substrate. The radiation imaging apparatus includes at least one radiation-sensitive imaging region in the substrate including a plurality of pixels (e.g., pixel electrodes), wherein each pixel of the plurality of pixels is configured to act as a detector element. The radiation imaging apparatus also includes a guard region at or immediately adjacent a cut edge of the substrate, wherein the guard region collects leakage current from the cut edge and reduces leakage current reaching the at least one radiation-sensitive imaging region from the cut edge when the radiation imaging apparatus is in use. The at least one radiation-sensitive imaging region is electrically reverse biased with respect to the substrate. The at least one radiation-sensitive imaging region includes a portion adjacent the guard region that is closest to where incident radiation impacts the radiation imaging apparatus. The plurality of pixel electrodes includes a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance.

In certain embodiments, the first distance is greater than 30 micrometers from the guard region. In certain embodiments, the first distance ranges between greater than 30 micrometers and 1000 micrometers. In certain embodiments, the first distance is 100 micrometers. In certain embodiments, the first distance reduces the amount of the leakage current received by the first pixel electrode relative to the pixel electrode by multiple fold (e.g., approximately five-fold when the first distance is 100 micrometers). In certain embodiments, the substrate includes a top cut edge, a bottom cut edge opposite the top cut edge, a first side cut edge, and a second side cut edge opposite the first side cut edge, wherein the first side cut edge and the second side cut edge extend between the top cut edge and the bottom cut edge, wherein the first distance is greater than a third distance, and wherein the third distance is a pixel-to-guard ring distance between any pixel electrode of plurality of pixels that is immediately adjacent to a portion of the guard region that is immediately adjacent to either the first side cut edge or the second side cut edge.

In certain embodiments, the plurality of pixels are arranged into a plurality of rows of pixel electrodes that are vertically arranged with respect to each other, and each pixel electrode in a top row of pixel electrodes located in the portion has a respective longitudinal end closest to the guard region located at the first distance from the guard region. In certain embodiments, the first pixel electrode comprises a third longitudinal end opposite the first longitudinal end that is located a same distance from the guard region as a fourth longitudinal end of the pixel electrode that is opposite the third longitudinal end, and the first pixel electrode is shorter along a longitudinal axis than the pixel electrode. In certain embodiments, the first pixel electrode is 1 to 2 percent shorter along the longitudinal axis relative to the pixel electrode.

In certain embodiments, the radiation imaging apparatus includes a photon counting X-ray detector. In certain embodiments, the photon counting detector is configured to be utilized with a computed tomography imaging system. In certain embodiments, the photon counting detector is configured to be utilized with a projectional radiographic imaging system.

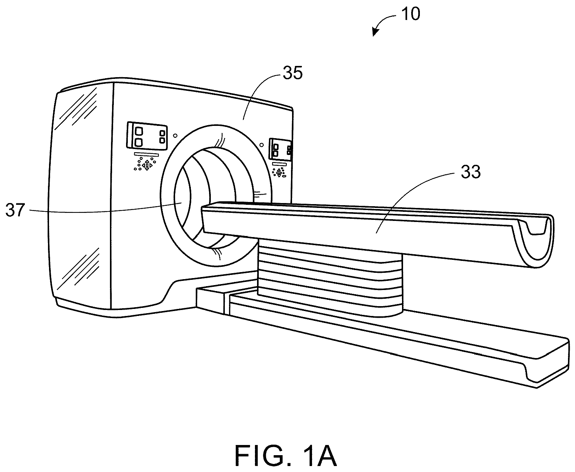

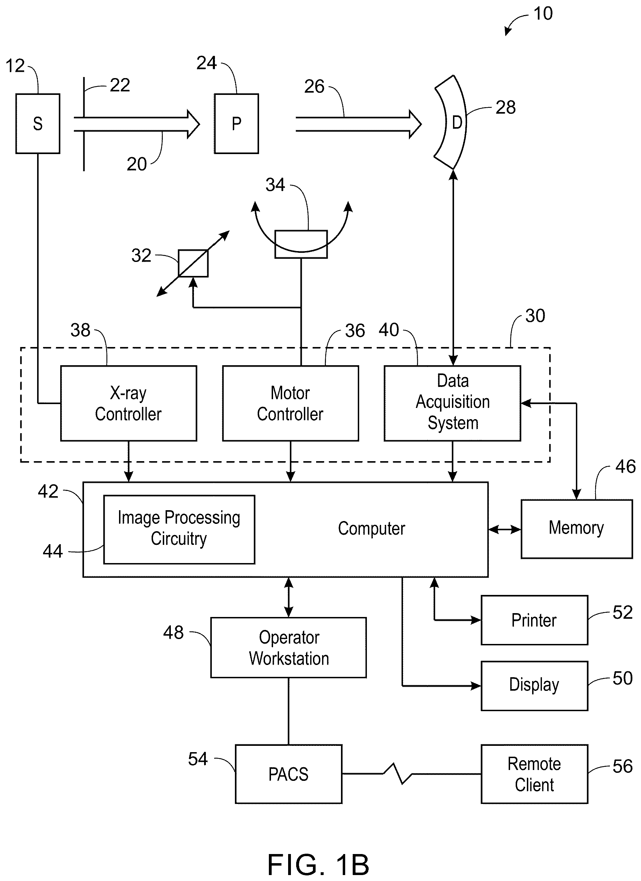

With the preceding discussion in mind, FIGS. 1A and 1B illustrate an embodiment of an imaging system 10 for acquiring and processing image data utilizing the techniques discussed herein. Although the following embodiments are discussed in terms of the computed tomography (CT) imaging system, the embodiments may also be utilized with other imaging systems (e.g., X-ray, PET, CT/PET, SPECT, nuclear CT, etc.). In particular, the disclosed detector may be utilized with a projectional (i.e., conventional) radiographic imaging system. In the illustrated embodiment, system 10 is a computed tomography (CT) system designed to acquire X-ray projection data, to reconstruct the projection data into a tomographic image, and to process the image data for display and analysis. The CT imaging system 10 includes one or more X-ray sources 12, such as one or more X-ray tubes or solid-state emission structures which allow X-ray generation at one or more locations and/or one or more energy spectra during an imaging session.

In certain implementations, the source 12 may be positioned proximate to a collimator 22 used to define the size and shape of the one or more X-ray beams 20 that pass into a region in which a subject 24 (e.g., a patient) or object of interest is positioned. The subject 24 attenuates at least a portion of the X-rays. Resulting attenuated X-rays 26 impact a detector array 28 formed by a plurality of detector elements (e.g., pixels). As discussed herein, the detector 28 may be a photon counting detector, including an energy-discriminating photon counting detector, whose outputs convey information about the number and energy of photons that impact the detector at measured positions and over a time interval corresponding to a scan or imaging session. In certain such embodiments, the energy-discriminating, photon counting detector may be a direct-conversion type detector (i.e., not employing a scintillator intermediary), such as a detector based on silicon strips. In certain embodiments, the detector array 28 may be formed by a plurality of detector sub-modules or sensors (each having a plurality of detector elements such as photodiode or diodes). In certain embodiments, the detector array 28 and the detector sub-modules may be an edge-on detector and edge-on detector sub-modules configured for edge illumination from the X-rays (i.e., the X-rays enter through the edge of the detector sub-modules).

Each detector element produces an electrical signal that represents the intensity of the incident X-ray photons (e.g., the energy and number of incident photons) at the position of the detector element when the beam strikes the detector 28. Electrical signals are acquired and processed to generate one or more scan datasets.

A system controller 30 commands operation of the imaging system 10 to execute examination and/or calibration protocols and to process the acquired data. With respect to the X-ray source 12, the system controller 30 furnishes power, focal spot location, control signals and so forth, for the X-ray examination sequences. The detector 28 is coupled to the system controller 30, which commands acquisition of the signals generated by the detector 28. In addition, the system controller 30, via a motor controller 36, may control operation of a linear positioning subsystem 32 (e.g., a table 33 in FIG. 1A) and/or a rotational subsystem 34 (e.g., a gantry 35 in FIG. 1A, a C-arm, etc.) used to move components of the imaging system 10 and/or the subject 24 (e.g., moving the subject 24 into and out of a bore or opening 37 of the gantry 35 in FIG. 1A) . The system controller 30 may include signal processing circuitry and associated memory circuitry. In such embodiments, the memory circuitry may store programs, routines, and/or encoded algorithms executed by the system controller 30 to operate the imaging system 10, including the X-ray source 12, and to process the data acquired by the detector 28 in accordance with the steps and processes discussed herein. In one embodiment, the system controller 30 may be implemented as all or part of a processor-based system such as a general purpose or application-specific computer system.

The source 12 may be controlled by an X-ray controller 38 contained within the system controller 30. The X-ray controller 38 may be configured to provide power and timing signals to the source 12. In addition, in some embodiments the X-ray controller 38 may be configured to selectively activate the source 12 such that tubes or emitters at different locations within the system 10 may be operated in synchrony with one another or independent of one another.

The system controller 30 may include a data acquisition system (DAS) 40. The DAS 40 receives data collected by readout electronics (e.g., ASICs) of the detector 28, such as sampled analog signals from the detector 28. The DAS 40 may then convert the data to digital signals for subsequent processing by a processor-based system, such as a computer 42. In other embodiments, the detector 28 may convert the sampled analog signals to digital signals prior to transmission to the data acquisition system 40. The computer may include processing circuitry 44 (e.g., image processing circuitry). The computer 42 may include or communicate with one or more non-transitory memory devices 46 that can store data processed by the computer 42, data to be processed by the computer 42, or instructions to be executed by a processor (e.g., processing circuitry 44) of the computer 42. For example, the processing circuitry 44 of the computer 42 may execute one or more sets of instructions stored on the memory 46, which may be a memory of the computer 42, a memory of the processor, firmware, or a similar instantiation.

The computer 42 may also be adapted to control features enabled by the system controller 30 (i.e., scanning operations and data acquisition), such as in response to commands and scanning parameters provided by an operator via an operator workstation 48. The system 10 may also include a display 50 coupled to the operator workstation 48 that allows the operator to view relevant system data, imaging parameters, raw imaging data, reconstructed data, and so forth. Additionally, the system 10 may include a printer 52 coupled to the operator workstation 48 and configured to print any desired measurement results. The display 50 and the printer 52 may also be connected to the computer 42 directly or via the operator workstation 48. Further, the operator workstation 48 may include or be coupled to a picture archiving and communications system (PACS) 54. PACS 54 may be coupled to a remote system 56, radiology department information system (RIS), hospital information system (HIS) or to an internal or external network, so that others at different locations can gain access to the image data.

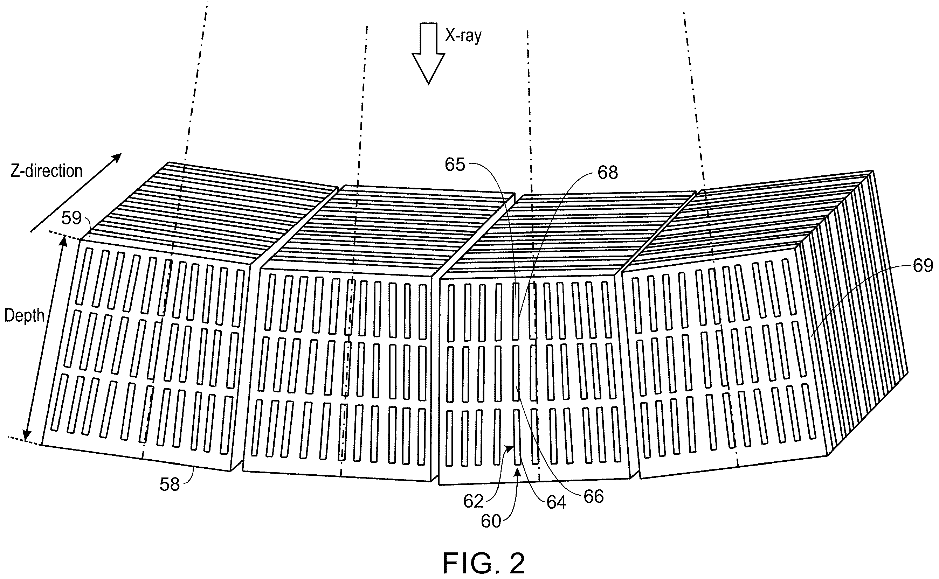

FIG. 2 is a schematic diagram illustrating an example of a modular X-ray detector sub-module 58 (e.g., detector sensors) arranged side-by-side and stacked one after the other. The detector sub-modules 58 may be edge-on detector sub-modules. As depicted, X-rays enter through an edge 59 of the detector sub-module 58. A guard ring (e.g., current capture guard electrode) may extend along the edges 59 of the detector sub-module 58 to protect the detector sub-module 58 from electrical breakdown and isolate the detector area from excessive leakage current. In certain embodiments, the detector sub-modules 58 may be planar modules. The X-ray detector sub-modules 58 may be stacked one after the other to form larger detector modules that may be assembled together side-by side to build up an overall X-ray detector. The detector sub-modules 58 may generally be arranged side-by-side, e.g., in a slightly curved overall configuration, in a direction substantially perpendicular to the z-direction. In certain embodiments, the detector sub-modules 58 may be stacked one after the other in the z-direction.

As depicted, each detector sub-module 58 includes a plurality of detector elements 60 (e.g., pixels or pixel electrodes such photodiodes or diodes). The detector elements 60 may be elongated electrodes (e.g., metal photodiode electrodes) with the length extension directed towards a focal point of an X-ray system. Depending on the detector topology, the detector element 60 may correspond to a pixel. In certain embodiments, the detector sub-module 58 may be a depth-segmented detector sub-module having a number of detector strips 62 with each strip 62 having a number of depth segments 65. As depicted, each strip 62 has a first segment 64, a second segment 66, and a third segment 68 associated with a different depth (relative to the focal point) along a detection line. As depicted, at least portions of each segment 64, 66, 68 are co-linearly arranged. The number of segments 65 may vary (e.g., 1 to 3 or more). For such a depth-segmented detector sub-module 58, each depth segment 65 may be regarded as an individual detector element (if each depth segment is associated with its own individual charge collecting electrode). In certain embodiments, circuitry may treat the depth segments 65 of a single strip 62 logically as a single detector element.

The shape of the detector sub-module 58 may vary. In certain embodiments, the detector sub-module 58 may have a parallelogram shape, a trapezoidal shape, a triangular shape, or another shape. In certain embodiments, one or more edges 59 of the detector sub-module 58 may be slanted. The shapes of the detector elements 60 may vary. In certain embodiments, the detector elements 60 arranged along a slanted side edge 69 of the detector sub-module 58 may include tapered edge segments (e.g., trapezoidal or triangular segments and/or truncated trapezoidal or triangular segments with rounded corners). In certain embodiments, a segment 65 of a strip 62 that is closest to a slanted side edge of a detector sub-module 58 may be orientated so that it extends into an area of an adjacent strip 62. In certain embodiments, the segments 65 may also be slanted.

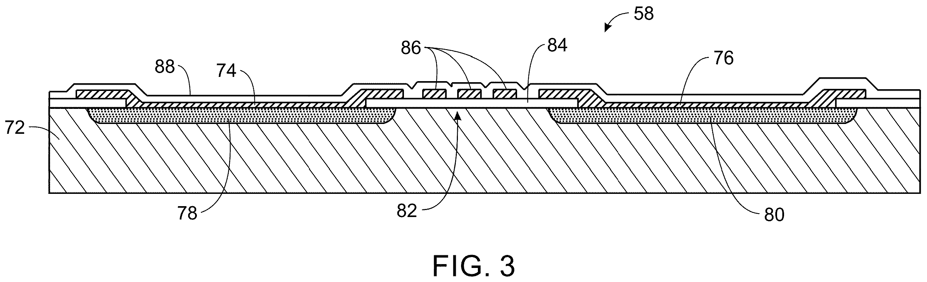

FIG. 3 is a cross-sectional view through a portion of the X-ray detector sub-module 58. The X-ray detector sub-module 58 includes a semiconductor layer 72. The semiconductor layer 72 is made of silicon. In certain embodiments, the semiconductor layer 72 may be made of gallium arsenide, cadmium zinc telluride, or another semiconductor material. Detector elements or segments 74, 76 (e.g., metal photodiode electrodes) are disposed on the semiconductor layer 72. The electrodes may be made of aluminum. In particular, the electrodes 74, 76 are disposed on doped implants 78, 80 (e.g., p-type or n-type silicon implants depending on whether the silicon of the semiconductor layer is n-type or p-type) that are disposed on the semiconductor layer 72. The detector elements 74, 76 may be respective segments for different strips of segments disposed adjacent to each other. The detector elements 74, 76 and the doped implants 78, 80 are disposed on the semiconductor layer 72 spaced apart so that a gap 82 is formed between them. The X-ray detector sub-module 58 includes an electrical insulator layer 84 extending between the adjacent electrodes 74, 76. The electrical insulator layer 84 may be silicon dioxide, silicon nitride, polyimide, spin-on glass, or another insulating material. One or more wiring traces 86 (e.g., metal traces) are routed within the gap 82 between the electrodes 74, 76. As depicted, the wiring traces 86 are disposed on the electrical insulator layer 84. As depicted, the wiring traces 86 are disposed in an evenly spaced manner across the gap 82. In certain embodiments, the wiring traces 86 may be routed as close as possible to the edges of the electrodes 74, 76. The wiring traces 86 may be coupled to the electrodes 74, 76 or different electrodes. The wiring traces 86 are routed along the gap 82 (and possibly other gaps) to readout circuitry. As depicted, a passivation layer 88 is disposed over these components of the X-ray detector sub-module 58. The passivation layer 88 may be made of silicon oxide, silicon nitride or another insulator.

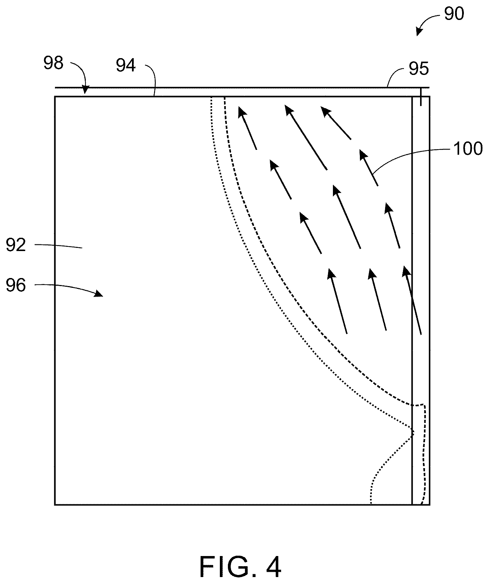

FIG. 4 is a schematic diagram of a sensor 90 (e.g., modular X-ray detector sub-module 58 in FIGS. 2 and 3) illustrating leakage current physics. As depicted, the sensor 90 includes a substrate 92 (e.g., semiconductor substrate or layer). A guard ring or guard region 94 (e.g., current capture guard electrode (CCR)) is disposed adjacent to and extends along edges 95 of the sensor 90 to protect the sensor 90 from electrical breakdown and isolate the detector area from excessive leakage current. The sensor 90 includes a radiation-sensitive imaging region 96 (e.g., active detector region) disposed within the guard ring 94 (which the guard ring 94 defines) and an inactive region 98 along the edges 95 outside the guard ring 94. The radiation-sensitive imaging region 96 is electrically reverse biased with respect to the substrate 92. Leakage current mainly originates from the diced edge 95 of the sensor 90. The internal electric field (determined by the surface charge and high voltage bias (reverse electrical bias)) drives the leakage carriers to the guard ring 94 and the edge pixels as indicated by arrows 100.

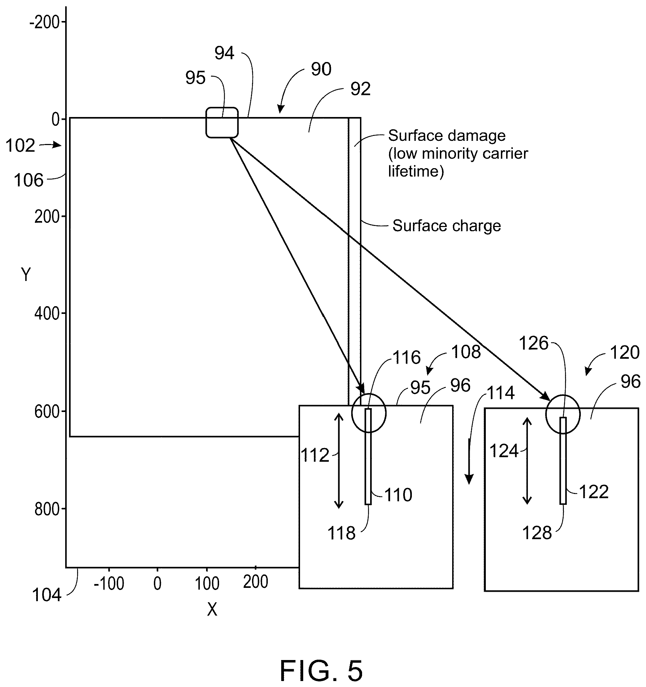

FIG. 5 is a schematic diagram of different configurations of the sensor 90 illustrating an alteration in pixel electrode position to reduce leakage current. As described in FIG. 4, the sensor 90 includes the substrate 92 (e.g., semiconductor substrate or layer). The guard ring 94 is disposed adjacent to and extends along the edges 95 of the sensor 90 to protect the sensor 90 from electrical breakdown and isolate the detector area from excessive leakage current. The sensor 90 includes the radiation-sensitive imaging region 96 disposed within the guard ring 94. The radiation-sensitive imaging region 96 is electrically reverse biased with respect to the substrate 92. Graph 102 depicts distances along the sensor 90. The graph 102 includes an x-axis 104 representing an x-direction along the sensor 90 and a y-axis 106 representing a y-direction along the sensor 90. In a typical configuration of the sensor 90 (e.g., sensor 108), a pixel electrode 110 (e.g., detector element 60 in FIG. 2) within the radiation-sensitive imaging region 96 has a longitudinal length 112 extending (e.g., in a vertical direction 114) between longitudinal ends 116 and 118 with the longitudinal end 116 disposed closest to the edge 95. The longitudinal end 116 is a distance from the edge 95 that results in increased current leakage to the pixel electrode 110. As described in greater detail herein, in a modified configuration of the sensor 90 (e.g., sensor 120), a pixel electrode 122 (e.g., detector element 60 in FIG. 2) within the radiation-sensitive imaging region 96 has a longitudinal length 124 extending (e.g., in the vertical direction 114) between longitudinal ends 126 and 128 with the longitudinal end 126 disposed closest to the edge 95. The longitudinal end 126 is a distance from the edge 95 (which is further from the edge than the longitudinal end 116 of the pixel electrode 110) that results in decreased current leakage to the pixel electrode 122. The farther the pixel electrode 122 is from the edge 95, the smaller the fraction of the leakage current that ends up in the pixel electrode 122. The pixel electrodes 110 and 122 are edge pixel electrodes since they are disposed adjacent to edge 95.

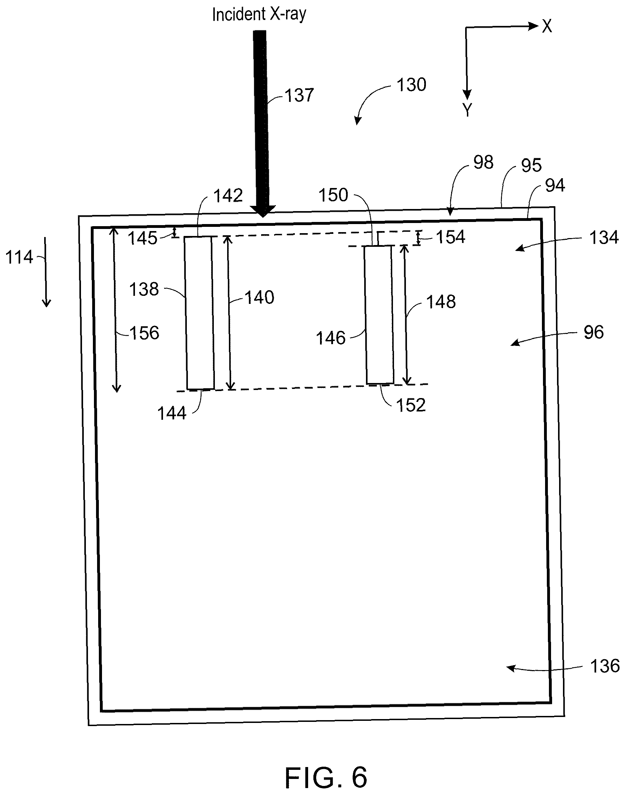

FIG. 6 is a schematic diagram of a sensor 130 comparing different pixel electrode positions. Sensor 130 (e.g., modular X-ray detector sub-module 58 in FIGS. 2 and 3) includes the substrate 92 (e.g., semiconductor substrate or layer). The guard ring 94 (e.g., current capture guard electrode (CCR)) is disposed adjacent to and extends along edges 95 of the sensor 130 to protect the sensor 130 from electrical breakdown and isolate the detector area from excessive leakage current. The sensor 130 includes the radiation-sensitive imaging region 96 (e.g., active detector region) disposed within the guard ring 94 (which the guard ring 94 defines) and the respective inactive region 98 along the edges 95 outside the respective guard ring 94. The radiation-sensitive imaging region 96 is electrically reverse biased with respect to the substrate 92 (via contacts coupled to the guard ring 94 and the radiation-sensitive imaging region 96). The radiation-sensitive imaging region 96 includes a portion 134 (e.g., top portion) and a portion 136 (e.g., bottom portion). Top and bottom are defined relative to the impact of incident radiation 137 on the sensor 130. The portion 134 is closest to (or adjacent to) where the incident radiation 137 impacts the sensor 130. As depicted, pixel electrodes longitudinally extend in a direction (e.g., vertical direction 114) along the direction of the incident radiation 137.

Sensor 130 depicts a pixel electrode 138 (e.g., detector element 60 in FIG. 2) in a typical position within the portion 134 of the radiation-sensitive imaging region 96. The pixel electrode 138 has a longitudinal length 140 extending (e.g., in the vertical direction 114) between longitudinal ends 142 and 144 with the longitudinal end 142 disposed closest to the guard ring 94. The longitudinal end 142 is a distance 145 (pixel edge-to-guard ring distance in the y-direction) from the guard ring 94 that results in increased current leakage to the pixel electrode 138.

Sensor 130 also depicts a pixel electrode 146 (e.g., detector element 60 in FIG. 2) in a modified position within the portion 134 of the radiation-sensitive imaging region 96. The pixel electrode 146 has a longitudinal length 148 extending (e.g., in the vertical direction 114) between longitudinal ends 150 and 152 with the longitudinal end 150 disposed closest to the guard ring 94. The longitudinal end 150 is a distance 154 (pixel edge-to-guard ring distance in the y-direction) from the guard ring 94 (which is further from the guard ring 94 than the longitudinal end 142 of the pixel electrode 138) that results in decreased current leakage to the pixel electrode 146 (relative to the pixel electrode 138). The pixel electrodes 138 and 146 are edge pixel electrodes since they are disposed adjacent to edge 95. In certain embodiments, a surface of the sensor 130 and the edges 95 of the sensor 130 are passivated to minimize surface leakage and defects.

The distance 154 is greater than the distance 145. The distance 145 is 30 micrometers or less. The distance 154 is greater than 30 micrometers. In certain embodiments, the distance 154 ranges between greater than 30 micrometers to 1000 micrometers. For example, the distance 154 may be 31, 35, 40, 45, 50, 55, 60, 65, 70, 75, 80, 85, 90, 95, or 100 micrometers or greater or any distance therebetween. In certain embodiments, the distance 154 is 100 micrometers. The farther the pixel electrode 146 is from the edge 95, the smaller the fraction of the leakage current that ends up in the pixel electrode 146.

The longitudinal length 148 of the pixel electrode 146 is shorter than the longitudinal length 140 of the pixel electrode 138. In certain embodiments, the longitudinal length 148 is approximately 1 to 2 percent shorter than the longitudinal length 140. The longitudinal end 152 of the pixel electrode 146 is located a same distance 156 from the guard ring 94 as the longitudinal end 144 of the pixel electrode 138 (i.e., thus the pixel electrode 146 is shorter along a longitudinal axis or length than the pixel electrode 138). In particular, a bottom of the pixel electrodes 138 and 146 are located at a same vertical location in the vertical direction 114.

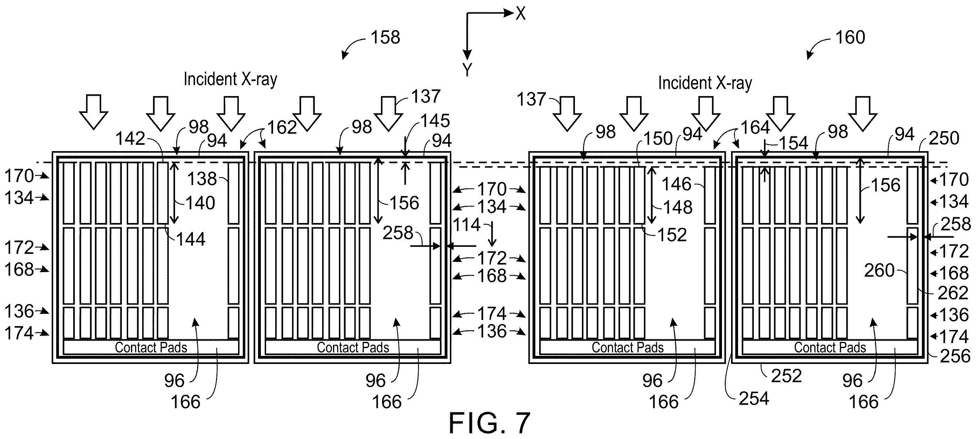

FIG. 7 is a schematic diagram comparing X-ray detectors having a top row of electrodes in a different positions relative to the guard ring 94. FIG. 7 depicts X-ray detectors 158 and 160. X-ray detector 158 has a plurality of sensors 162 (e.g., detector sub-modules 58 in FIG. 2) and X-ray detector 164 has a plurality of sensors 164 (e.g., detector sub-modules 58 in FIG. 2).

Each sensor 162, 164 includes a respective substrate 92 (e.g., semiconductor substrate or layer). Each sensor 162 and 164 includes a respective guard ring 94 (e.g., current capture guard electrode (CCR)) that is disposed adjacent to and extends along edges 95 of the respective sensor 162, 164 to protect the sensor 162, 164 from electrical breakdown and isolate the detector area from excessive leakage current. Each sensor 162, 164 includes the radiation-sensitive imaging region 96 (e.g., active detector region) disposed within the guard ring 94 (which the guard ring 94 defines) and the respective inactive region 98 along the edges 95 outside the respective guard ring 94. The radiation-sensitive imaging region 96 is electrically reverse biased with respect to the substrate 92 (via contacts (coupled to contacts pads 166) coupled to the guard ring 94 and the radiation-sensitive imaging region 96). The radiation-sensitive imaging region 96 includes a portion 134 (e.g., top portion), a portion 168 (e.g., middle portion), and a portion 136 (e.g., bottom portion). Top and bottom is defined relative to the impact of incident radiation 137 on the respective sensor 162, 164. The portion 134 is closest to (or adjacent to) where the incident radiation 137 impacts the respective sensor 162, 164 (and respective X-ray detector 158, 160). As depicted, pixel electrodes longitudinally extend in a direction (e.g., vertical direction 114 or y-direction) along the direction of the incident radiation 137.

Each sensor 162 includes a plurality of pixel electrodes 138 (e.g., detector elements 60 in FIG. 2) arranged in a plurality of rows that are vertically arranged (in vertical direction 114) with respect to each other. As depicted, there are three rows of pixel electrodes 138. In certain embodiments, the number of rows may vary. As depicted, each sensor 162 includes a top row 170 of pixel electrodes 138 in the portion 134, a middle row 172 of pixel electrodes 138 in the portion 168, and a bottom row 174 of pixel electrodes 138 in the portion 136. The top row 170 of pixel electrodes 138 are depicted in a typical position within the portion 134 of the radiation-sensitive imaging region 96. Each pixel electrode 138 in the top row 170 has a longitudinal length 140 extending (e.g., in the vertical direction 114) between longitudinal ends 142 and 144 with the longitudinal end 142 disposed closest to the guard ring 94. The longitudinal end 142 is a distance 145 (pixel edge-to-guard ring distance in the y-direction) from the guard ring 94 that results in increased current leakage to the top row 170 of pixel electrodes 138.

Each sensor 164 includes a plurality of pixel electrodes 146 (e.g., detector elements 60 in FIG. 2) arranged in a plurality of rows that are vertically arranged (in vertical direction 114) with respect to each other. As depicted, there are three rows of pixel electrodes 146. In certain embodiments, the number of rows may vary. As depicted, each sensor 164 includes a top row 170 of pixel electrodes 146 in the portion 134, a middle row 172 of pixel electrodes 146 in the portion 168, and a bottom row 174 of pixel electrodes 146 in the portion 136. The top row 170 of pixel electrodes are depicted in a modified position within the portion 134 of the radiation-sensitive imaging region 96. Each pixel electrode 146 in the top row 170 has a longitudinal length 148 extending (e.g., in the vertical direction 114) between longitudinal ends 150 and 152 with the longitudinal end 150 disposed closest to the guard ring 94. The longitudinal end 150 (for each pixel electrode 146 in the top row 170) is a distance 154 (pixel edge-to-guard ring distance in the y-direction) from the guard ring 94 (which is further from the guard ring 94 than the longitudinal end 142 of the pixel electrode 138) that results in decreased current leakage to the top row 170 of pixel electrodes 146 (relative to the top row 170 of pixel electrodes 138). In certain embodiments, a respective surface of the sensors 162, 164 and the edges 95 of the sensors 162, 164 are passivated to minimize surface leakage and defects.

The distance 154 is greater than the distance 145. The distance 145 is 30 micrometers or less. The distance 154 is greater than 30 micrometers. In certain embodiments, the distance 154 ranges between greater than 30 micrometers to 1000 micrometers. For example, the distance 154 may be 31, 35, 40, 45, 50, 55, 60, 65, 70, 75, 80, 85, 90, 95, or 100 micrometers or greater or any distance therebetween. In certain embodiments, the distance 154 is 100 micrometers. The farther the top row 170 of pixel electrodes 146 is from the edge 95, the smaller the fraction of the leakage current that ends up in the top row 170 of pixel electrodes 146.

The longitudinal length 148 of the pixel electrodes 146 in the top row 170 of the sensors 164 is shorter than the longitudinal length 140 of the pixel electrode 138 in the top row 170 of the sensors 162. In certain embodiments, the longitudinal length 148 is approximately 1 to 2 percent shorter than the longitudinal length 140. The longitudinal end 152 of the pixel electrode 146 in the top row 170 is located a same distance 156 from the guard ring 94 as the longitudinal end 144 of the pixel electrode 138 in the top row 170 (i.e., thus the pixel electrode 146 is shorter along a longitudinal axis or length than the pixel electrode 138). In particular, a bottom of the pixel electrodes 138 and 146 in the respective top rows 170 are located at a same vertical location in the vertical direction 114. The pixel electrodes 138 in the middle row 172 and the bottom row 174 of the sensors 162 are the same with respect to geometry and location to the pixel electrodes 146 in the middle row 172 and the bottom row 174 of the sensors 164.

The substrate 92 includes a top cut edge 250, a bottom cut edge 252 opposite the top cut edge 250, a first side cut edge 254, and a second side cut edge 256 opposite the first side cut edge 254. The first side cut edge 254 and the second side cut edge 256 extend between the top cut edge 250 and the bottom cut edge 252 in the y-direction, wherein the distance 154 is greater than a pixel-to-guard ring distance 258 in the x-direction between any pixel electrode (e.g., pixel electrode 260) of the plurality of pixel electrodes 146 that is immediately adjacent to a portion of the guard ring 94 that is immediately adjacent to either the first side cut edge 254 or the second side cut edge 256. The pixel-to-guard ring distance 258 is between the guard ring 92 and a lateral edge of the pixel electrode (e.g., lateral edge 262 of the pixel electrode 260) nearest the guard ring 92. In certain embodiments, the pixel-to-guard ring distance 258 is 30 micrometers. The pixel-to-guard ring distance 258 and the distance 145 are the same with the sensors 162.

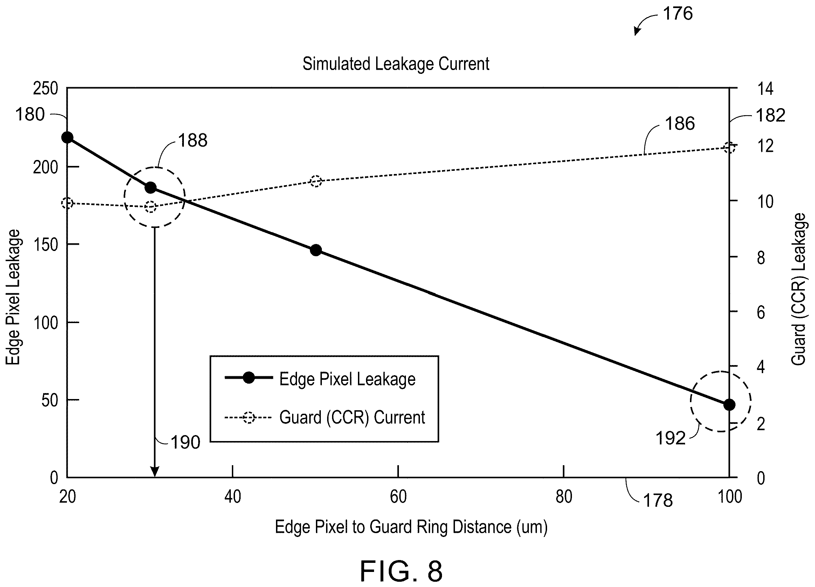

FIG. 8 is a table 176 depicting simulated leakage current for increasing pixel electrode to guard ring distance. The table 176 includes an x-axis 178 representing a distance between a guard ring and a longitudinal end of a top row pixel electrode closest to the guard ring adjacent the location where incident radiation first interfaces with a sensor. The table 176 also includes a left y-axis 180 representing edge pixel leakage and a right y-axis 182 representing guard ring linkage. Plot 184 represents edge pixel leakage. Plot 186 represents guard ring current. The data was generated with a technology computer-aided design device simulation. Dashed circle 188 indicates the edge pixel leakage when the edge pixel electrode of the top row has its longitudinal end closest to the guard ring at a typical distance of 30 micrometers (as indicated by arrow 190). Dashed circle 192 indicates the edge pixel leakage when the edge pixel electrode of the top row has its longitudinal end closest to the guard ring at a modified distance of 100 micrometers. As depicted, as the distance increases between the longitudinal end of the edge pixel electrode, the current leakage of the edge pixel electrode decreases. When the distance is 100 micrometers, the current leakage of the edge pixel electrode is reduced approximately five-fold relative to the current leakage of the edge pixel electrode at the typical distance (e.g., 30 micrometers).

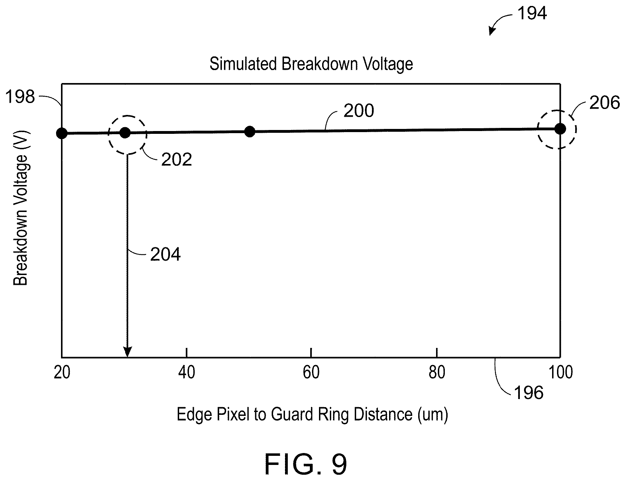

FIG. 9 is a table 194 depicting simulated breakdown voltage for increasing pixel electrode to guard ring distance. The table 194 includes an x-axis 196 representing a distance between a guard ring and a longitudinal end of a top row pixel electrode closest to the guard ring adjacent the location where incident radiation first interfaces with a sensor. The table 194 also includes a y-axis representing breakdown voltage 198. Plot 200 represents breakdown voltage of the edge pixel electrode. The data was generated with a technology computer-aided design device simulation. Dashed circle 202 indicates the breakdown voltage when the edge pixel electrode of the top row has its longitudinal end closest to the guard ring at a typical distance of 30 micrometers (as indicated by arrow 204). Dashed circle 206 indicates the breakdown voltage when the edge pixel electrode of the top row has its longitudinal end closest to the guard ring at a modified distance of 100 micrometers. As depicted, increasing the distance between the guard ring and the longitudinal end of the top row pixel electrode closest to the guard ring does not impact detection efficiency and does not impact sensor reliability (as impact on breakdown voltage is negligible as depicted in the table 194).

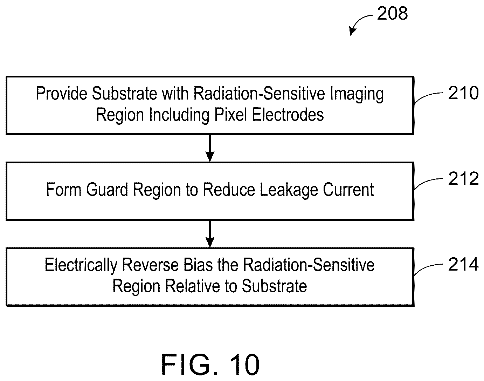

FIG. 10 is a flow chart of a method 208 of reducing leakage current in a radiation imaging apparatus. The method 208 includes providing a substrate with at least one radiation-sensitive imaging region therein, wherein the at least one radiation-sensitive imaging region includes a plurality of pixels, wherein each pixel of the plurality of pixels is configured to act as a detector element (block 210). The method 208 also includes forming a guard region in the substrate at or immediately adjacent a cut edge of the substrate to collect leakage current from the cut edge and to reduce the leakage current reaching the at least one radiation-sensitive imaging region from the cut edge when the radiation imaging apparatus is in use (block 212). The method 208 further includes electrically reverse biasing the at least one radiation-sensitive imaging region relative to the substrate (block 214). The at least one radiation-sensitive imaging region includes a portion adjacent the guard region that is closest to where incident radiation impacts the radiation imaging apparatus. The plurality of pixels includes a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance. The pixel electrodes in the portion may be formed utilizing a modified mask from the typical mask.

Technical effects of the disclosed embodiments include reducing leakage current in an X-ray detector. In particular, pixels (e.g., diodes) at the edge of the sensor (e.g., detector sub-module) closest to interaction with incident radiation, which are typically prone to high leakage, are shortened (along their longitudinal length) so that these edge pixels are farther away from a current capture guard electrode to reduce an amount of the leakage current received by each of these edge pixels. Technical effects of the disclosed embodiments include improving sensor yield resulting in detector cost savings. Technical effects of the disclosed embodiments include reducing dark counts due to a lower leakage current and thus improve image quality.

The disclosure also provides support for a radiation imaging apparatus, comprising: a substrate; at least one radiation-sensitive imaging region in the substrate comprising a plurality of pixels, wherein each pixel of the plurality of pixels is configured to act as a detector element; a guard region at or immediately adjacent a cut edge of the substrate, wherein the guard region collects leakage current from the cut edge and reduces the leakage current reaching the at least one radiation-sensitive imaging region from the cut edge when the radiation imaging apparatus is in use; and wherein the at least one radiation-sensitive imaging region is electrically reverse biased with respect to the substrate, wherein the at least one radiation-sensitive imaging region comprises a portion adjacent the guard region that is closest to where incident radiation impacts the radiation imaging apparatus, wherein the plurality of pixels comprises a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance. In a first example of the radiation imaging apparatus, the first distance is greater than 30 micrometers from the guard region. In a second example of the radiation imaging apparatus, optionally including the first example, the first distance ranges between greater than 30 micrometers and 1000 micrometers. In a third example of the radiation imaging apparatus, optionally including one or both of the first and second examples, the first distance is 100 micrometers. In a fourth example of the radiation imaging apparatus, optionally including one or more or each of the first through third examples, the substrate comprises a top cut edge, a bottom cut edge opposite the bottom cut edge, a first side cut edge, and a second cut edge opposite the first side cut edge, wherein the first side cut edge and the second cut edge extend between the top cut edge and the bottom cut edge, wherein the first distance is greater than a third distance, and wherein the third distance is a pixel-to-guard ring distance between any pixel electrode of plurality of pixels that is immediately adjacent to a portion of the guard region that is immediately adjacent to either the first side cut edge or the second cut edge. In a fifth example of the radiation imaging apparatus, optionally including one or more or each of the first through fourth examples, the plurality of pixels are arranged into a plurality of rows of pixel electrodes that are vertically arranged with respect to each other, and each pixel electrode in a top row of pixel electrodes located in the portion has a respective longitudinal end closest to the guard region located at the first distance from the guard region. In a sixth example, optionally including one or more or each of the first through fifth examples, the first pixel electrode comprises a third longitudinal end opposite the first longitudinal end that is located a same distance from the guard region as a fourth longitudinal end of the pixel electrode that is opposite the third longitudinal end, and the first pixel electrode is shorter along a longitudinal axis than the pixel electrode. In a seventh example, optionally including one or more or each of the first through sixth examples, the first pixel electrode is 1 to 2 percent shorter along the longitudinal axis relative to the pixel electrode. In an eighth example, optionally including one or more or each of the first through seventh examples, the radiation imaging apparatus comprises a photon counting X-ray detector. In a ninth example, optionally including one or more or each of the first through eighth examples, the photon counting detector is configured to be utilized with a computed tomography imaging system. In a tenth example, optionally including one or more or each of the first through ninth examples, the photon counting detector is configured to be utilized with a projectional radiographic imaging system.

The disclosure also provides support for a method of reducing leakage current in a radiation imaging apparatus, comprising: providing a substrate with at least one radiation-sensitive imaging region therein, wherein the at least one radiation-sensitive imaging region comprises a plurality of pixels, wherein each pixel of the plurality of pixels is configured to act as a detector element; forming a guard region in the substrate at or immediately adjacent a cut edge of the substrate to collect leakage current from the cut edge and to reduce the leakage current reaching the at least one radiation-sensitive imaging region from the cut edge when the radiation imaging apparatus is in use; and electrically reverse biasing the at least one radiation-sensitive imaging region relative to the substrate, wherein the at least one radiation-sensitive imaging region comprises a portion adjacent the guard region that is closest to where incident radiation impacts the radiation imaging apparatus, wherein the plurality of pixels comprises a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance. In a first example of the method, the first distance is greater than 30 micrometers from the guard region. In a second example of the method, optionally including the first example, the first distance ranges between greater than 30 micrometers and 1000 micrometers. In a third example of the method, optionally including one or both of the first and second examples, the first distance is 100 micrometers. In a fourth example of the method, optionally including one or more or each of the first through third examples, the substrate comprises a top cut edge, a bottom cut edge opposite the bottom cut edge, a first side cut edge, and a second cut edge opposite the first side cut edge, wherein the first side cut edge and the second cut edge extend between the top cut edge and the bottom cut edge, wherein the first distance is greater than a third distance, and wherein the third distance is a pixel-to-guard ring distance between any pixel electrode of plurality of pixels that is immediately adjacent to a portion of the guard region that is immediately adjacent to either the first side cut edge or the second cut edge. In a fifth example of the method, optionally including one or more or each of the first through fourth examples, the plurality of pixels are arranged into a plurality of rows of pixel electrodes that are vertically arranged with respect to each other, and each pixel electrode in a top row of pixel electrodes located in the region has a respective longitudinal end closest to the guard region located at the first distance from the guard region. In a sixth example of the method, optionally including one or more or each of the first through fifth examples, the radiation imaging apparatus comprises a photon counting X-ray detector.

The disclosure also provides support for a photon-counting X-ray detector, comprising: a plurality of detector sub-modules, wherein each detector sub-module comprises: a semiconductor layer; at least one radiation-sensitive imaging region in the semiconductor layer, wherein the at least one radiation-sensitive imaging region comprises a plurality of pixels, wherein each pixel of the plurality of pixels is configured to act as a detector element; a guard region at or immediately adjacent a cut edge of the semiconductor layer, wherein the guard region collects leakage current from the cut edge and reduces the leakage current reaching the at least one radiation-sensitive imaging region from the cut edge when the radiation imaging apparatus is in use; and wherein the at least one radiation-sensitive imaging region is electrically reverse biased with respect to the semiconductor layer, wherein the at least one radiation-sensitive imaging region comprises a portion adjacent the guard region that is closest to where incident radiation impacts the radiation imaging apparatus, wherein the plurality of pixels comprises a first pixel electrode located in the portion where a first longitudinal end of the first pixel electrode closest to the guard region is a first distance from the guard region that reduces an amount of the leakage current received by the first pixel electrode relative to if a pixel electrode were located in the portion having a second longitudinal end closest to the guard region located at a second distance from the guard region that is less than the first distance. In a first example of the photon-counting X-ray detector, the first distance ranges between greater than 30 micrometers and 1000 micrometers.

The techniques presented and claimed herein are referenced and applied to material objects and concrete examples of a practical nature that demonstrably improve the present technical field and, as such, are not abstract, intangible or purely theoretical. Further, if any claims appended to the end of this specification contain one or more elements designated as "means for [perform]ing [a function]..." or "step for [perform]ing [a function]...", it is intended that such elements are to be interpreted under 35 U.S.C. 112(f). However, for any claims containing elements designated in any other manner, it is intended that such elements are not to be interpreted under 35 U.S.C. 112(f).

This written description uses examples to disclose the present subject matter, including the best mode, and also to enable any person skilled in the art to practice the subject matter, including making and using any devices or systems and performing any incorporated methods. The patentable scope of the subject matter is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A radiation imaging apparatus (90), comprising:
a substrate (92);
at least one radiation-sensitive imaging region (96) in the substrate (92) comprising a plurality of pixels (60) , wherein each pixel (60) of the plurality of pixels (60) is configured to act as a detector element;
a guard region (94) at or immediately adjacent a cut edge (95) of the substrate (92), wherein the guard region (94) collects leakage current from the cut edge (95) and reduces the leakage current reaching the at least one radiation-sensitive imaging region (96) from the cut edge (95) when the radiation imaging apparatus (90)is in use; and
wherein the at least one radiation-sensitive imaging region (90) is reverse biased with respect to the substrate (92), wherein the at least one radiation-sensitive imaging region (96) comprises a portion (134) adjacent the guard region (94) that is closest to where incident radiation impacts the radiation imaging apparatus (90), wherein the plurality of pixels (60) comprises a first pixel electrode (146) located in the portion (134) where a first longitudinal end (150) of the first pixel electrode (146) closest to the guard region (94) is a first distance (154) from the guard region (94) that reduces an amount of the leakage current received by the first pixel electrode (146) relative to if a pixel electrode (138) were located in the portion (134) having a second longitudinal end (142) closest to the guard region (94) located at a second distance (145) from the guard region (94) that is less than the first distance (154).

2. The radiation imaging apparatus (90) of claim 1, wherein the first distance (154) is greater than 30 micrometers from the guard region (94).

3. The radiation imaging apparatus (90) of claim 2, wherein the first distance (154) ranges between greater than 30 micrometers and 1000 micrometers.

4. The radiation imaging apparatus (90) of claim 3, wherein the first distance (154) is 100 micrometers.

5. The radiation imaging apparatus (90) of claim 1, wherein the substrate (72) comprises a top cut edge (250), a bottom cut edge (252) opposite the top cut edge (250), a first side cut edge (254), and a second side cut edge (256) opposite the first side cut edge (254), wherein the first side cut edge (254) and the second side cut edge (256) extend between the top cut edge (250) and the bottom cut edge (252), wherein the first distance (154) is greater than a third distance(258), and wherein the third distance (258) is a pixel-to-guard ring distance between any pixel electrode of plurality of pixels (60) that is immediately adjacent to a portion of the guard region (94) that is immediately adjacent to either the first side cut edge (254) or the second side cut edge (256).

6. The radiation imaging apparatus (90) of claim 1, wherein the plurality of pixels (60) are arranged into a plurality of rows of pixel electrodes (146) that are vertically arranged with respect to each other, and each pixel electrode (146) in a top row (170) of pixel electrodes (146) located in the portion (134) has a respective longitudinal end (150) closest to the guard region (94) located at the first distance (154) from the guard region (94).

7. The radiation imaging apparatus (90) of claim 1, wherein the first pixel electrode (146) comprises a third longitudinal end (152) opposite the first longitudinal end (150) that is located a same distance (156) from the guard region (94) as a fourth longitudinal end (144) of the pixel electrode(138) that is opposite the third longitudinal end (142), and the first pixel electrode (146) is shorter along a longitudinal axis (148) than the pixel electrode (138).

8. The radiation imaging apparatus (90) of claim 7, wherein the first pixel electrode (146) is 1 to 2 percent shorter along the longitudinal axis (148) relative to the pixel electrode (138).

9. The radiation imaging apparatus (90) of claim 1, wherein the radiation imaging apparatus (96) comprises a photon counting X-ray detector.

10. The radiation imaging apparatus (90) of claim 9, wherein the photon counting X-ray detector is configured to be utilized with a computed tomography imaging system (10).

11. The radiation imaging apparatus (90) of claim 9, wherein the photon counting X-ray detector is configured to be utilized with a projectional radiographic imaging system.

12. A method of reducing leakage current in a radiation imaging apparatus (90), comprising:
providing a substrate (92) with at least one radiation-sensitive imaging region (96) therein, wherein the at least one radiation-sensitive imaging region (96) comprises a plurality of pixels (60), wherein each pixel (60) of the plurality of pixels (60) is configured to act as a detector element;
forming a guard region (94) in the substrate (92) at or immediately adjacent a cut edge (95) of the substrate (92) to collect leakage current from the cut edge (95) and to reduce the leakage current reaching the at least one radiation-sensitive imaging region (96) from the cut edge (95) when the radiation imaging apparatus (96) is in use; and
electrically reverse biasing the at least one radiation-sensitive imaging region (96) relative to the substrate (92), wherein the at least one radiation-sensitive imaging region (96) comprises a portion (134) adjacent the guard region (94) that is closest to where incident radiation impacts the radiation imaging apparatus (90), wherein the plurality of pixels (60) comprises a first pixel electrode (146) located in the portion (134) where a first longitudinal end (150) of the first pixel electrode (146) closest to the guard region (96) is a first distance (154) from the guard region (94) that reduces an amount of the leakage current received by the first pixel electrode (146) relative to if a pixel electrode (138) were located in the portion (134) having a second longitudinal end (142) closest to the guard region (94) located at a second distance (145) from the guard region (94) that is less than the first distance (154).

13. The method of claim 12, wherein the first distance (154) is greater than 30 micrometers from the guard region (94).

14. The method of claim 12, wherein the first distance (154) ranges between greater than 30 micrometers and 1000 micrometers.

15. The method of claim 14, wherein the first distance (154) is 100 micrometers.
